# FASCICULE DE BREVET EUROPEEN

(11) **EP 0 632 838 B1**
(45) Date de publication et mention de la délivrance du brevet: **12.12.2001**
(21) Numéro de dépôt: 94905147.8
(22) Date de dépôt: 24.01.1994
(51) Int. Cl.: C12Q 1/04, C12P 7/52, C12Q 1/18

(54) **MILIEU SELECTIF ET PROCEDE POUR LE DENOMBREMENT DES BACTERIES PROPIONIQUES**
SELEKTIVES MEDIUM UND VERFAHREN ZUR ZÄHLUNG VON PROPIONISCHEN BAKTERIEN
SELECTIVE MEDIUM AND METHOD FOR ENUMERATING PROPIONIC BACTERIA

(30) Priorité: 27.01.1993 FR 9300823
(43) Date de publication de la demande: 11.01.1995
(73) Titulaire: INSTITUT NATIONAL DE LA RECHERCHE AGRONOMIQUE, F-75341 Paris Cédex 07 (FR)
(72) Inventeur: MADEC, Marie-No[lle, F-35000 Rennes (FR); ROUAULT, Annette, F-35000 Rennes (FR); MAUBOIS, Jean-Louis, F-35740 Pace (FR); THIERRY, Anne, F-35850 Romille (FR)
(74) Mandataire: Phélip, Bruno
(86) Numéro de dépôt international: FR9400082
(87) Numéro de publication internationale: WO9417201

(56) Documents cités:
- EP-A- 0 174 477
- FR-A- 2 328 771
- US-A- 4 714 675
- US-A- 5 026 647
- JOURNAL OF DAIRY SCIENCE vol. 51, no. 10 , 1968 pages 1707 - 1709 D.H.HETTINGA ET AL. 'Pouch Method for Isolating and Enumerating Propionibacteria' cité dans la demande
- APPLIED AND ENVIRONMENTAL MICROBIOLOGY vol. 38, no. 4 , Octobre 1979 pages 585 - 589 M.KISHISHITA ET AL. 'Biotyping of Propionibacterium acnes Isolated from Normal Human Facial Skin.'
- "The Merck Index, An Encyclopedia of Chemicals, Drugs and Biologicals", 12th edition, Merck & Co. Inc., 1996.

## Description

La présente invention a pour objet un milieu sélectif pour le dénombrement des bactéries propioniques.

Elle est également relative à un procédé pour le dénombrement de ces microorganismes.

Les bactéries propioniques ont un rôle essentiel dans l'obtention des caractéristiques organoleptiques spécifiques des fromages à pâte pressée cuite (Emmental-Gruyère-Comté ). Elles interviennent dans la production de CO₂ à l'origine de la formation des "yeux", dans la production d'acide acétique et surtout propionique à l'origine de la saveur et dans la dégradation peptidasique des dérivés des protéines laitières à l'origine de la flaveur.

Ce genre bactérien a été aussi utilisé dans un passé récent pour la production industrielle de vitamine B₁₂. D'autres emplois sont actuellement proposés : production de propionates en bioréacteur (brevet français n° 90 04 985 publication 2.660.932) lesdits propionates étant utilisés pour leurs propriétés antifongiques en panification et en fromagerie , et production de biomasse pour une utilisation en tant que probiotique ( alimentation de la vache laitière ; conservation d'ensilage).

Que ce soit pour ces utilisations ou pour la détermination précise du rôle joué dans les processus d'affinage des fromages à pâte pressée , le progrès des connaissances sur les bactéries propioniques passe par l'existence d'un moyen de dénombrement spécifique, c'est-à-dire d'un milieu de culture sélectif.

Un tel milieu sélectif n'existe pas à la connaissance du demandeur.

Le milieu de référence, couramment utilisé dans l'industrie fromagère, est le YELA. (Yeast Extract Lactate Agar) décrit par Hettinga et al., 1968 ( J. Dairy Science, 51 1707-1709). Ce milieu permet de dénombrer la flore propionique lorsque celle-ci est majoritaire . Il n'est en aucun cas inhibiteur des bactéries lactiques mésophiles et thermophiles qui constituent la flore dominante des fromages à pâte pressée cuite .

Deux autres milieux basés sur la limitation des nutriments azotés ( remplacement de l'extrait de levure par du sulfate d'ammonium ou un hydrolysat trypsique de caséine: trypticase) et l'apport corrélatif en quantités notables de vitamines (biotine, pantothénate de calcium, acide para amino benzoïque et thiamine) et de sels minéraux essentiels ( Mg⁺⁺; Mn⁺⁺ et Fe⁺⁺ notamment) ont été décrits par Peberdy et Fryer (NZ J. Dairy Science and Techn., 11, 10-15, 1976). Ces milieux dénommés A.S.L.A. ( Ammonium Sulphate Lactate Agar) et T.L.A. (Trypticase Lactate Agar), s'ils présentent une sélectivité relative, nécessitent au moins 10 jours d'incubation en anaérobiose et de plus doivent être utilisés en parallèle pour dénombrer les bactéries propioniques présentes dans les fromages . Ces limitations d'emploi ont été considérées comme non acceptables par les utilisateurs potentiels et l'emploi des milieux ASLA et TLA n'a connu aucun développement.

On a aussi proposé l'addition de cadmium ou d'un mélange de sels d'arsenic et d'un antibiotique, la Netilmicine, à un milieu lactate agar ( brevet US 5.026.647).

Mais ces milieux ne permettent la croissance que d'un nombre limité de souches appartenant à l'espèce P. jensenii et apparaissent inhibiteurs pour les autres espèces de bactéries propioniques.

De plus, il est évident qu'une utilisation généralisée de ces milieux créerait de sérieux risques de toxicité tant pour les manipulateurs que pour l'environnement du fait du rejet d'arsenic et de cadmium.

Les bactéries propioniques laitières sont connues pour être résistantes à la plupart des sulfamides, à certaines pénicillines du groupe des pénicillines-M, telle que l'oxacilline ou la cloxacilline, ainsi qu'à l'acide nalidixique ( Reddy et al; 1972, J. Dairy Sci 55,665; 1973, J. Milk Food Technol 30, 564-569; 1973, Antimicrob Ag Chemother 4,254-258). Ces mêmes auteurs ont montré que les souches testées de Propionibacterium présentaient également une résistance modérée à des polypeptides (colistine et polymixine B) et à des antibiotiques du groupe des aminosides (néomycine et kanamycine). Les bactéries propioniques sont en revanche sensibles à la plupart des β-lactamines (pénicilline G et A, céfalosporines) ainsi qu'aux cyclines (tétracycline), à des macrolides tels l'érythromycine et au chloramphénicol. (Reddy et al., 1973, J. Milk Food Technol. 30, 564-569; Nord and Olsson-Liljequist, 1985 J. Antimicrob. Chemother, 15, suppl. C, 183-188). En ce qui concerne les souches cutanées de Propionibacterium, l'utilisation dans le traitement de l'acné, d'antibiotiques du groupes des macrolides (érythromycine), du groupe des lincosamides (lincomycine) et du groupe des cyclines (tétracycline) a entraîné l'apparition de souches résistantes et a nécessité la mise en oeuvre d'autres antibiotiques (Eady et al., 1989, J. Antimicrob Chemother, 23, 493-502; Eady et al. 1993,Br J. Dermatol 128, 556-560; Kurasawa et al, 1988, J. Dermatol 15, 149-154). Un milieu sélectif a été proposé pour l'isolement des souches sauvages et résistantes aux antibiotiques de Propionibacterium acnes (Marino and Stoughton, 1982 J. American Acad. Dermatol, 6, 902-908).

Les travaux de Reddy et al. (1972 et 1973, précédemment cités) ont été ultérieurement utilisés dans le brevet US 5.026.647 et dans les travaux de Drinan et Cogan (1992, J. Dairy Research, 59, 65-69) visant à proposer des milieux sélectifs pour le dénombrement des bactéries propioniques laitières.

Ainsi, Cogan et Drinan ( 1992 ) ont proposé d'ajouter un antibiotique, la cloxacilline au milieu SLA (Sodium Lactate Agar). Cet antibiotique inhibe la croissance des ferments lactiques présents dans le fromage mais aux dires mêmes des auteurs, n'inhibe pas la croissance des lactobacilles mésophiles, des entérocoques et des Clostridium, microorganismes fréquemment rencontrés dans les fromages.

L'addition d'acide nalidixique (0,02% p/v) à un milieu contenant de l'extrait de levure, du lactate de sodium et de l'agar a été utilisée récemment pour rechercher les bactéries propioniques dans du fromage Leerdammer et dans des réacteurs anaérobies (Riedel et Britz,( 1993)Biodiversity and Conservation 2, 400-411). Dans les deux cas, ce milieu se révèle insuffisamment sélectif pour permettre le dénombrement des bactéries propioniques.

Enfin, la demande FR-2.328.771 décrit un milieu de détermination de staphylocoques contenant, entre autres constituants, du chlorure de lithium, de la pimaricine et du D-mannitol.

Il ressort de l'état de la technique porté à la connaissance du demandeur qu'aucun milieu ne permet un dénombrement spécifique des bactéries propioniques.

Le demandeur s'est donc attaché à mettre au point un milieu permettant un dénombrement spécifique et relativement rapide des bactéries propioniques à partir d'un échantillon biologique.

Il a ainsi montré de manière surprenante qu'un milieu de culture contenant du lithium ainsi que du glycérol ou de l'erythritol, et/ou des antibiotiques permet un dénombrement spécifique des bactéries propioniques.

La présente invention a donc pour objet un milieu pour le dénombrement en anaérobiose des bactéries propioniques caractérisé en ce qu'il comprend un milieu complexe complémenté avec d'une part au moins un composé de lithium et d'autre part du glycérol ou de l'érythritol et/ou un ou plusieurs antibiotiques choisis parmi la fosfomycine, des aminosides, des polypeptides, des quinolones de première et de seconde générations et des imidazoles, auxquels les bactéries propioniques sont résistantes.

On entend par milieu complexe un milieu comprenant au moins une source multiple de nutriments telle qu'un extrait de levure, un hydrolysat de caséine ou une autre peptone.

Cette ou ces sources contiennent une forte proportion d'acides aminés et de petits peptides , ainsi que des vitamines , des sucres , des bases puriques ou pyrimidiques et d'autres facteurs de croissance des microorganismes .

Préférentiellement, le milieu complexe servant de base à ce milieu de dénombrement est composé de tryptone et d'extrait de levure, par exemple ceux commercialisés par la Société BIOKAR.

Un tel milieu , du fait de la présence d'un composé de lithium, inhibe le développement des bactéries lactiques mésophiles et thermophiles sans inhiber la croissance des bactéries propioniques. Ces bactéries peuvent par contre utiliser et biotransformer en acide propionique les polyols contenus dans le milieu .

Le composé de lithium est préférentiellement compris dans le milieu entre 0,2 % et 2 % en poids du milieu et le polyol est préférentiellement compris entre 0,01 % et 2 % en poids du milieu. Un tel milieu est avantageusement utilisé sous une forme gélosée. Il comprend préférentiellement comme composé de lithium du lactate de lithium, de l'hydroxyde de lithium ou du chlorure de lithium.

Si le milieu contient du lactate de lithium, la concentration de ce sel peut être comprise entre 2 et 20 g/l, préférentiellement entre 5 et 15 g/l et encore plus préférentiellement est d'environ 10 g/l.

Le milieu objet de la présente invention peut contenir, outre le composé de lithium, soit uniquement des polyols, soit uniquement un ou plusieurs antibiotiques, soit un mélange de polyols et d'antibiotiques.

Le polyol contenu dans ce milieu est du glycérol ou de l'érythritol.

Avantageusement le milieu comprend de 0,1 à 20 g/l, préférentiellement de 2 à 10 g/l et encore plus préférentiellement environ 6 g/l de glycérol.

Lorsque la flore propionique est minoritaire, par exemple dans le lait ou les fromages fabriqués à partir de lait cru ou thermisé, la différenciation des bactéries propioniques sur un milieu selon l'invention ne contenant que des polyols va se révéler difficile.

En effet, sur un milieu contenant des polyols mais ne contenant pas d'antibiotiques, la révélation de la présence de colonies de bactéries propioniques va être faite grâce à la formation d'une zone de virage de l'indicateur coloré autour de ces colonies, dus à la fermentation du polyol. Dans le cas de l'utilisation du pourpre de bromocrésol, par exemple, les colonies de bactéries propioniques apparaissent entourées d'un halo jaune sur un fond violet.

Lorsque la flore adventice est en quantité importante, la totalité du milieu de culture vire au violet pâle et le critère de différenciation (décoloration du violet au jaune autour des colonies) n'est pas utilisable. Ceci est notamment vrai dans le cas du dénombrement des bactéries propioniques " sauvages" du lait cru.

Dans certains cas, il est donc nécessaire d'accroître la sélectivité de ce milieu. Ceci peut avantageusement être effectué par addition d'antibiotiques. Dans le cas où l'apport d'un ou plusieurs antibiotiques rend le milieu totalement sélectif, l'addition au milieu d'un polyol associé à un colorant pour différencier les bactéries propioniques n'est plus indispensable.

Le milieu selon l'invention comprend avantageusement un ou plusieurs antibiotiques appartenant aux groupes suivants (définis selon la dénomination commune internationale):
- fosfomycine
- aminosides (gentamicine, kanamycine, tobramycine,...)
- polypeptides ( colistine, polymixine B, ....)
- quinolones de première génération (acide nalidixique,...)
- quinolones de seconde génération (péfloxacine, norfloxacine, ...)
- imidazolés ( métronidazole).
- pivmecillinam

Les concentrations de chaque antibiotique sont avantageusement comprises entre 2 et 500 mg/l.

Pour le dénombrement des bactéries propioniques du lait, le mélange d'antibiotiques comprendra préférentiellement de la fosfomycine (2 à 100 mg/l), un antibiotique du groupe des aminosides (2 à 20 mg/l) et une quinolone de première génération (2 à 300 mg/l).

Les antibiotiques sont dilués dans un solvant approprié, stérilisés par un moyen adapté et ajoutés à la concentration requise au milieu de culture stérilisé.

D'une manière surprenante, l'addition d'un tel cocktail d'antibiotiques au milieu YELA ne permet pas le dénombrement des bactéries propioniques du lait. Bien que le mécanisme n'en soit pas élucidé, il apparaît que la présence simultanée du cocktail d'antibiotiques et du sel de lithium soit nécessaire pour permettre une sélectivité suffisante et une bonne croissance des bactéries propioniques.

On a également constaté que la croissance des bactéries propioniques sur le milieu selon l'invention est accélérée par l'addition de 1% de lait au milieu et permet ainsi de dénombrer les bactéries propioniques après 6 jours d'incubation. Le milieu selon la présente invention comprend donc préférentiellement entre 0,05 % et 5 % de lait.

On notera que, à la connaissance du demandeur, la résistance des bactéries propioniques laitières à la fosfomycine, au métronidazole, au Pivmecillinam ainsi qu'aux quinolones de seconde génération telles que la péfloxacine et la norfloxacine, n'avait jamais été décrite dans l'état de la technique.

La présente invention est, en outre, relative à un procédé de dénombrement de bactéries dans un échantillon biologique par incubation en anaérobiose dudit échantillon ou de ses dilutions décimales dans le milieu précédemment décrit et comptage desdites bactéries, par toute méthode connue de l'homme du métier .

Pour la mise en oeuvre de la présente invention, on se référera, si besoin est, au manuel suivant : GUIRAUD J. GALZY P. (1980). L'analyse microbiologique dans les industries alimentaires. Ed. L'Usine Nouvelle, Paris.

La présente invention est illustrée sans pour autant être limitée par les exemples qui suivent :

### EXEMPLE 1:

### Préparation d'un milieu de dénombrement contenant du glycérol.

A 1000 ml d'eau distillée, on ajoute :
- 10 g de tryptone commercialisée par la Société BIOKAR
- 10 g d'extrait de levure commercialisé par la Société BIOKAR
- 10 g de lactate de lithium commercialisé par la Société MERCK
- 0,25 g de PO₄HK₂ ( hydrogénophosphate de potassium), 0,05 g de SO₄Mn(sulfate de manganèse) commercialisés par la Société Merck et 0,05 g de pourpre de bromocrésol, commercialisé par la Société Sigma.

D'autres indicateurs colorés de variation de pH tels le bleu de bromothymol, le vert de bromocrésol ou le rouge de méthyle ou de phénol peuvent également être utilisés en lieu et place du pourpre de bromocrésol.

La solution obtenue est additionnée de 6 g de glycérol puis ajustée à pH 7,0 par addition de NaOH et gélosée par ajout de 15 g d'agar type E ( produit commercialisé par la Société Biokar) . Elle est portée à ébullition sous agitation pour dissoudre l'agar, est répartie en flacons et est stérilisée par autoclavage 20 minutes à 115°C.

### EXEMPLE 2:

### Dénombrement de bactéries sur le milieu de l'exemple 1.

### a) Comparaison du milieu de dénombrement selon l'invention avec le milieu YELA.

L'aptitude des bactéries propioniques appartenant aux 4 espèces retrouvées en milieu laitier, à se développer sur le milieu de culture faisant l'objet de la présente invention a été testée en comparaison avec le milieu de référence YELA.

Le mode opératoire d'utilisation du milieu selon l'invention est le suivant :
Le milieu gélosé obtenu dans l'exemple 1 a été refroidi à 46°C. 1 ml des cultures obtenues en 48 heures sur milieu YEL ( milieu YELA sans agar ) , ainsi que des dilutions décimales desdites cultures, ont été transférés dans des boîtes de Pétri stériles et ont été recouverts de 12 ml de milieu. Après homogénéisation soigneuse et solidification sur une surface froide, les boîtes ont été incubées 5 à 6 jours en anaérobiose à 30°C.

Les bactéries propioniques se présentent sous forme de colonies lenticulaires ou rondes de diamètre égal ou supérieur à 0,5 mm entourées d'une zone d'acidification de 1 à 5 mm de coloration jaune sur fond violet.

L'aptitude de 42 souches de bactéries propioniques (tableau 1), appartenant à 4 espèces différentes, à se développer sur le milieu de culture faisant l'objet de la présente invention a été testée en comparaison avec le milieu de culture de référence YELA.

On constate que le milieu de culture faisant l'objet de la présente invention conduit à des valeurs de dénombrement de cultures de bactéries propioniques tout à fait proches sinon identiques à celles obtenues avec le milieu de référence YELA.

### b) Dénombrement de bactéries de diverses espèces.

La sélectivité du milieu faisant l'objet de la présente invention a été testée vis-à-vis de 18 espèces bactériennes (tableau 2) représentatives des flores lactique et contaminante du lait ou de ses dérivés et vis-à-vis de 6 mélanges commerciaux de levains lactiques mésophiles et thermophiles.

1 ml des cultures de 48 h des différentes espèces à tester et des dilutions décimales desdites cultures sont transférés dans des boîtes de Pétri stériles. Le mode opératoire est ensuite le même que décrit précédemment.

### c) Dénombrement dans du lait.

La sélectivité du milieu de culture faisant l'objet de la présente invention a été testée vis-à-vis du milieu de référence YELA sur du lait ultra-propre ( lait écrémé cru microfiltré sur membrane ayant un diamètre de pores de 1,4 microns) additionné de 500 ml d'une culture de bactéries propioniques à 2,6.10⁹ cellules/ml pour 15.000 l de lait ( ce qui correspond à un ensemencement d'environ 1 x 10⁵ cellules de bactéries propioniques par ml de lait). Ledit lait a été également ensemencé avec 1 x 10⁶ bactéries lactiques mésophiles et thermophiles par ml. Les dénombrements réalisés avec les deux milieux ont conduit aux résultats suivants :
- milieu YELA : 1,7 x 10⁶ UFC/ml
- milieu selon l'invention : 4,5 x 10⁵ UFC/ml

Le milieu selon l'invention conduit à un dénombrement de bactéries propioniques très voisin de celui résultant de l'addition de la culture pure au lait alors que le milieu YELA donne une valeur près de 4 fois supérieure.

### d) Dénombrement dans des fromages à pâte pressée.

La sélectivité du milieu de culture faisant l'objet de la présente invention a également été testée sur différents fromages à pâte pressée . Pour ce faire, 10 g de fromage ont été ajoutés à 90 ml d'eau citratée ( solution de 20g de citrate trisodique dans 1000 ml d'eau distillée ajustée à pH 7,5 par addition d'hydroxyde de sodium). La suspension a ensuite été broyée 3 minutes au Stomacher puis a servi à l'ensemencement des milieux selon l'invention et YELA comme décrit précédemment. Les résultats obtenus sont rassemblés dans le tableau 3 ci-après.

### EXEMPLE 3 :

### Résistance des bactéries propioniques aux antibiotiques

Les cinq souches-types de bactéries propioniques laitières ont été testées quant à leur résistance à 27 antibiotiques. Ces tests ont été effectués au moyen de galeries ATB (BioMérieux, Marcy-l'Etoile, France) après culture de 48 heures sur bouillon YEL et dilution appropriée selon les indications du fournisseur. L'incubation des galeries a été effectuée à 30°C en anaérobiose pendant 30 heures. La croissance des bactéries propioniques a été évaluée de manière semi-quantitative à l'observation d'un trouble plus ou moins prononcé dans chaque cupule (tableau 4).

Ce tableau fait apparaître notamment une très bonne résistance des souches mises en oeuvre aux aminosides testés, à la fosfomycine, aux quinolones de 1ère et 2ème générations ainsi qu'à la colistine, au métronidazole et à l'oxacilline.

### EXEMPLE 4:

### Préparation du milieu de dénombrement contenant des antibiotiques.

Trois solutions d'antibiotiques sont préparées par addition dans de l'eau distillée des quantités suivantes :
solution F : fosfomycine (Sigma P5396) 256,0 mg qsp 50 ml eau distillée,
solution G: gentamicine (Sigma P3632) 32,0 mg qsp 100 ml eau distillée,
solution N: acide nalidixique (Sigma N 8878) 64,0 mg qsp 20 ml eau distillée en milieu basique (addition de NaOH).

Les solutions F, G, N ainsi que de l'eau distillée sont mélangées dans des proportions 5:10:2:23. Le mélange est ensuite filtré stérilement, réparti en tubes stériles et stocké à -20°C.

Au moment de l'utilisation, le mélange d'antibiotiques d'un tube-stock est décongelé. Cinq millilitres de ce mélange sont ajoutés à 95 ml du milieu gélosé stérile selon l'exemple 1, après refroidissement de celui-ci à 46°C. Le facteur de dilution dû à l'addition de la solution d'antibiotiques est pris en compte au moment de la préparation du milieu gélosé (préparé dans 950 ml avec les quantités suffisantes pour un litre).

### EXEMPLE 5:

### Dénombrement des bactéries propioniques en culture pure sur le milieu selon l'exemple 4.

Vingt-quatre souches de Propionibacterium appartenant aux cinq espèces laitières ont été testées quant à leur aptitude à se développer sur le milieu de culture de l'exemple 4, en comparaison avec leur croissance sur le milieu YELA. Les dénombrements ont été effectués après culture de 48 heures sur bouillon YEL. Un millilitre des dilutions décimales appropriées de ces cultures ont été transférées en boîtes de Pétri stériles. Le mode opératoire est ensuite le même que celui décrit dans l'exemple 2. Les résultats sont présentés dans le tableau 5.

L'écart entre les valeurs de dénombrement sur YELA et sur le milieu selon l'invention est en moyenne de 0,2 logarithme décimal.

Deux tiers des souches testées présentaient, après six jours d'incubation, des colonies d'aspect typique (diamètre des colonies supérieur ou égal à 0,5 mm et présence d'une zone de décoloration de 1 à 5 mm autour des colonies), ou répondant à l'un de ces deux critères. L'allongement de la durée d'incubation à 8 jours permet d'observer les deux critères de reconnaissance (diamètre des colonies et zone de décoloration) pour la quasi-totalité des souches testées.

### EXEMPLE 6:

### Effet de l'addition de lait aux milieux de culture selon les exemples 1 et 4.

L'addition de lait au milieu de culture additionné d'antibiotiques selon l'invention améliore la vitesse de croissance et permet de réduire le temps d'incubation nécessaire pour observer les caractéristiques typiques des colonies de bactéries propioniques.

Le tableau 6 décrit les résultats obtenus avec la souche Propionibacterium freudenreichii subsp. shermanii CIP 103027.

Le dénombrement a été effectué après culture de 48 heures comme décrit dans l'exemple 5.

L'addition de 0,1 % ou 1% de lait écrémé autoclavé a été effectuée dans le milieu de culture selon l'exemple 4 avant transfert en boîtes de Pétri. Les résultats sont présentés dans le tableau 6.

L'addition de 1% de lait se traduit par l'obtention de colonies de taille égale ou supérieure à la taille des colonies obtenues sur le milieu YELA et favorise par ailleurs l'obtention de colonies présentant après 6 jours d'incubation une zone de décoloration marquée.

### EXEMPLE 7:

### Dénombrement comparatif des bactéries propioniques du lait sur le milieu YELA et les milieux des exemples 1 et 4.

La sélectivité du milieu additionné d'antibiotiques faisant l'objet de la présente invention a été testée en comparaison avec le même milieu sans antibiotique et le milieu de référence YELA.

Pour 21 échantillons de lait de grand mélange, 1 ml de lait et des dilutions décimales desdits échantillons sont transférés dans des boites de Pétri stériles. Le mode opératoire est ensuite le même que celui décrit dans l'exemple 2.

Le tableau 7 rapporte les résultats des dénombrements des bactéries propioniques de lait de grand mélange au moyen du milieu YELA, du milieu selon l'exemple 1 ( milieu lithium-glycérol) et du milieu selon l'exemple 4 ( milieu lithium-glycérol-antibiotiques). Pour chaque milieu, deux comptages distincts ont été effectués après 6 jours d'incubation en anaérobiose:
1. le comptage de la totalité des microorganismes capables de se développer sur ce milieu;
2. le comptage des colonies de bactéries propioniques, dénombrées d'après les critères suivants:
   - sur YELA et milieu lithium-glycérol, colonies lenticulaires ou rondes de couleur crème de diamètre supérieur ou égal à 0,5 mm,
   - sur milieu lithium-glycérol-antibiotiques, colonies lenticulaires ou rondes de diamètre supérieur ou égal à 0,5 mm entourées d'une zone d'acidification de 1 à 5 mm jaune sur fond violet.

Ce tableau met en évidence l'accroissement de sélectivité apporté par l'addition d'antibiotiques au milieu lithium.

la différence entre la flore propionique et la totalité de la flore capable de se développer sur le milieu lithium-glycérol-antibiotiques est en moyenne de 0,10 logarithme décimal.

### EXEMPLE 8:

### Effet combiné du lithium et des antibiotiques.

Un échantillon de lait de grand mélange a été dénombré de manière comparative:
- sur le milieu YELA additionné ou non de lithium et/ou d'antibiotiques dans les mêmes proportions que celles détaillées dans l'exemple 4,
- sur le milieu selon l'exemple 1 ( milieu lithium-glycérol),
- sur le milieu selon l'exemple 4 (milieu lithium-glycérol-antibiotiques).

Le mode opératoire est identique à celui mis en oeuvre dans l'exemple 7. Les résultats sont consignés dans le tableau 8.

Il apparaît clairement que l'addition simultanée de lithium et du cocktail d'antibiotiques utilisé permet d'obtenir une sélectivité maximale, alors qu'aucune colonie typique n'a pu être dénombrée sur le milieu YELA additionné du seul cocktail d'antibiotiques.

De par sa sélectivité et son électivité, le milieu de culture faisant l'objet de la présente invention peut permettre :
1) de réaliser l'isolement rapide de nouvelles souches de bactéries propioniques qui peuvent ensuite être caractérisées sur leurs aptitudes technologiques ( production de CO₂, d'acide propionique, de vitamine B12);
2) de déterminer la flore propionique endogène des laits collectés en vue d'ajuster si besoin est, son niveau;
3) de suivre le développement de la flore propionique en fromagerie de pâte pressée.

En conclusion l'utilisation de ce milieu permet d'améliorer la qualité globale des fabrications fromagères à pâte pressée ou d'autres nouvelles variétés de fromages.

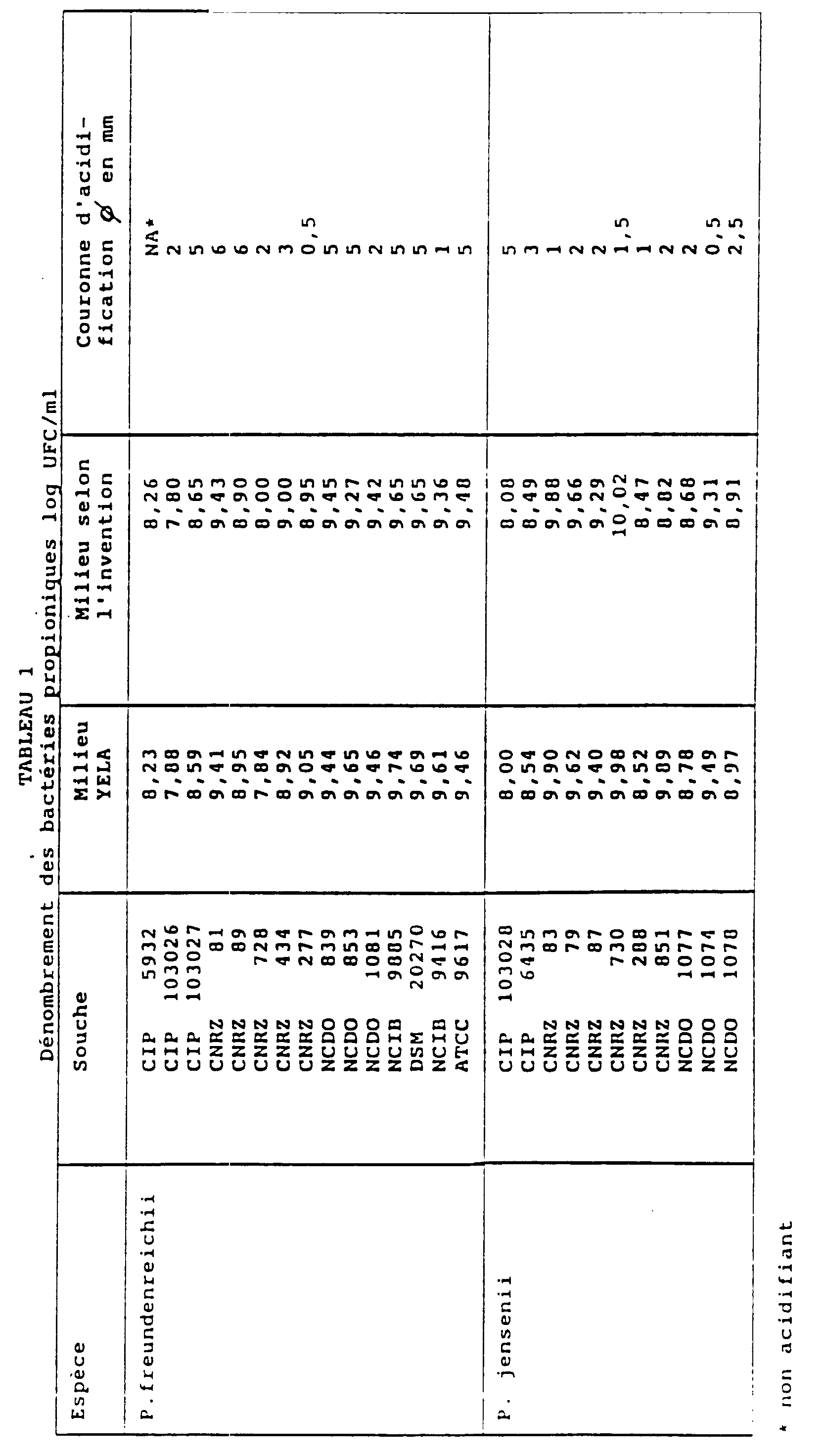

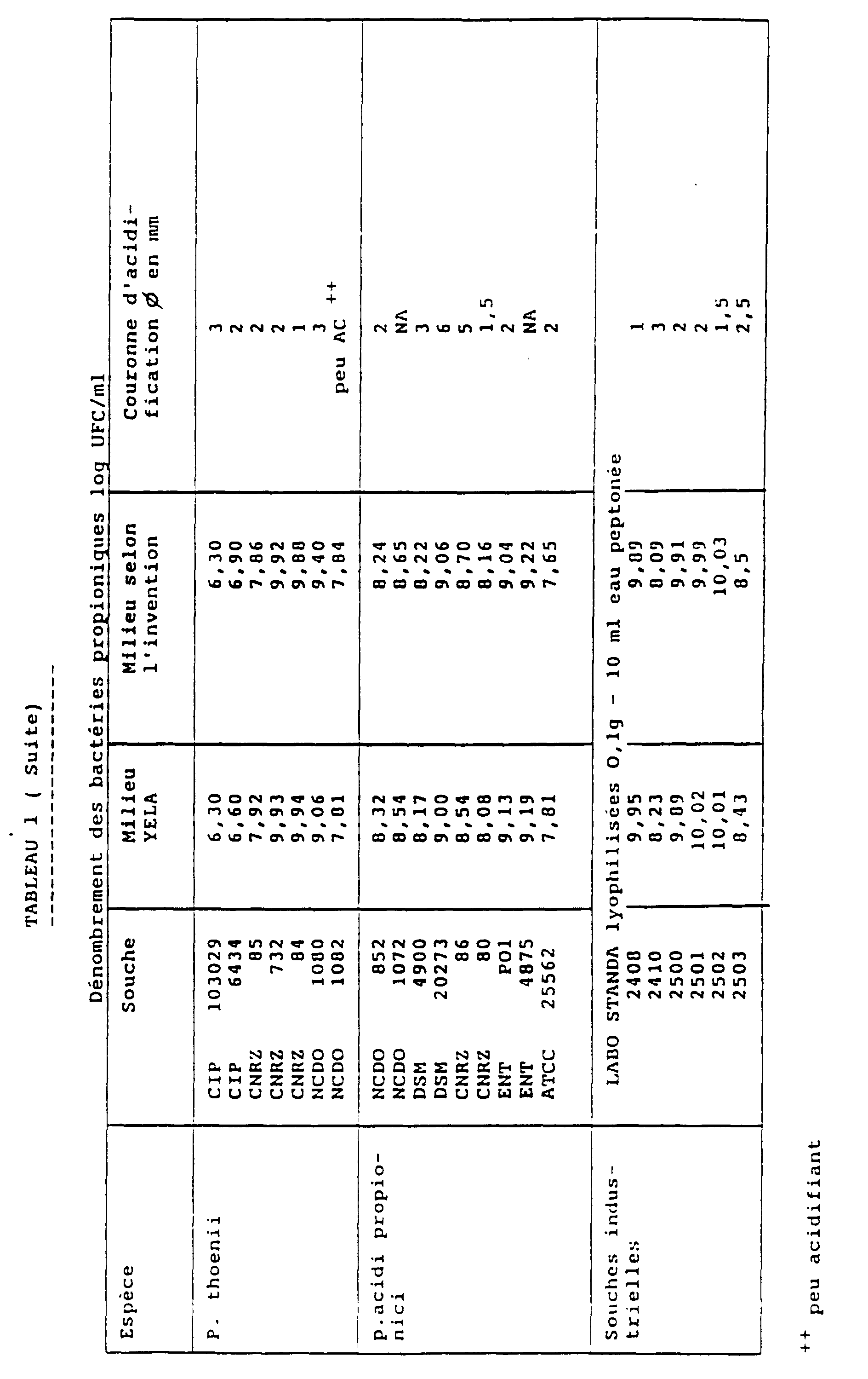

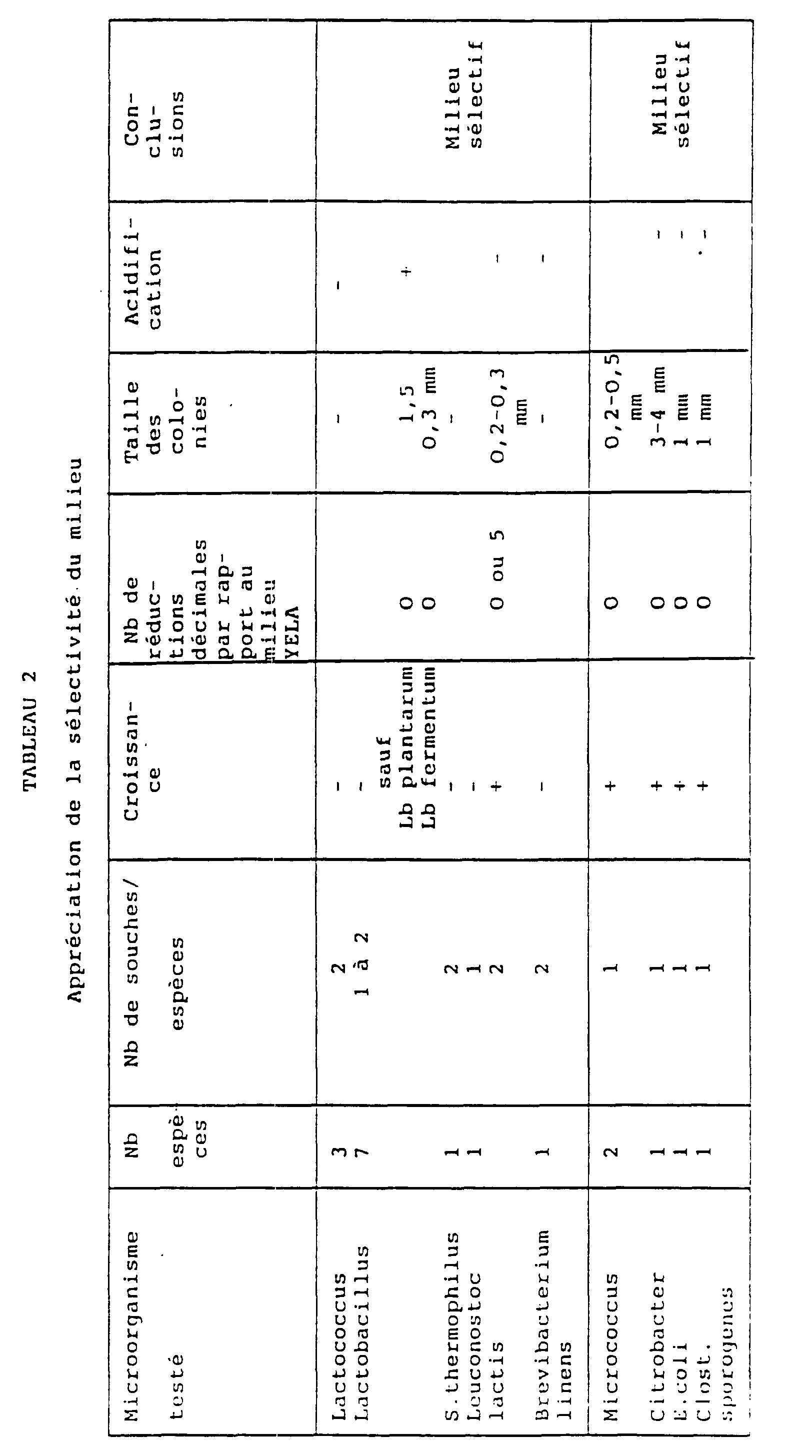

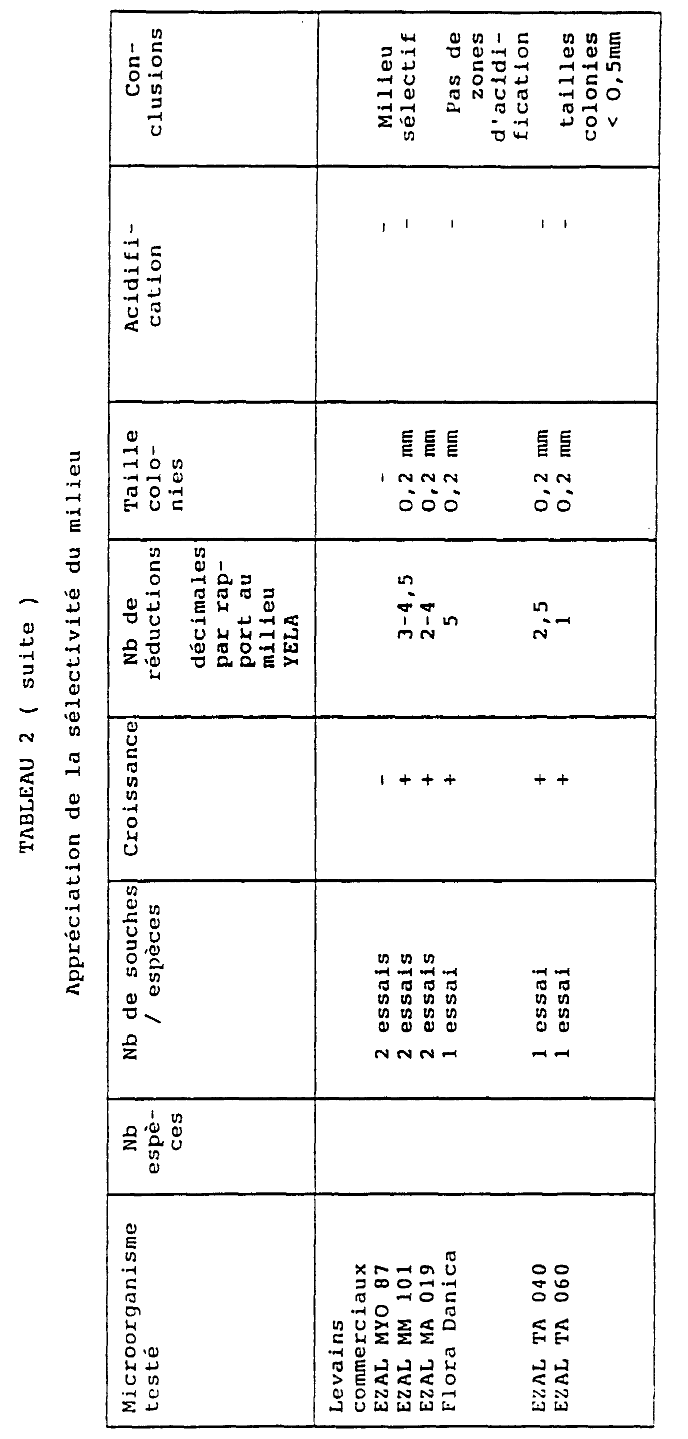

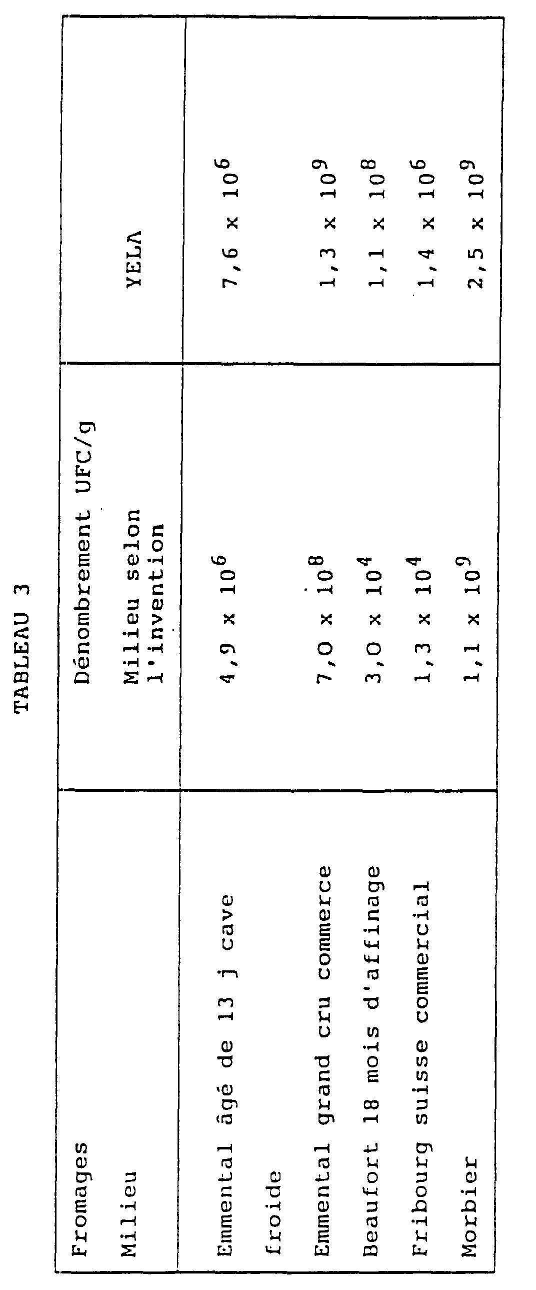

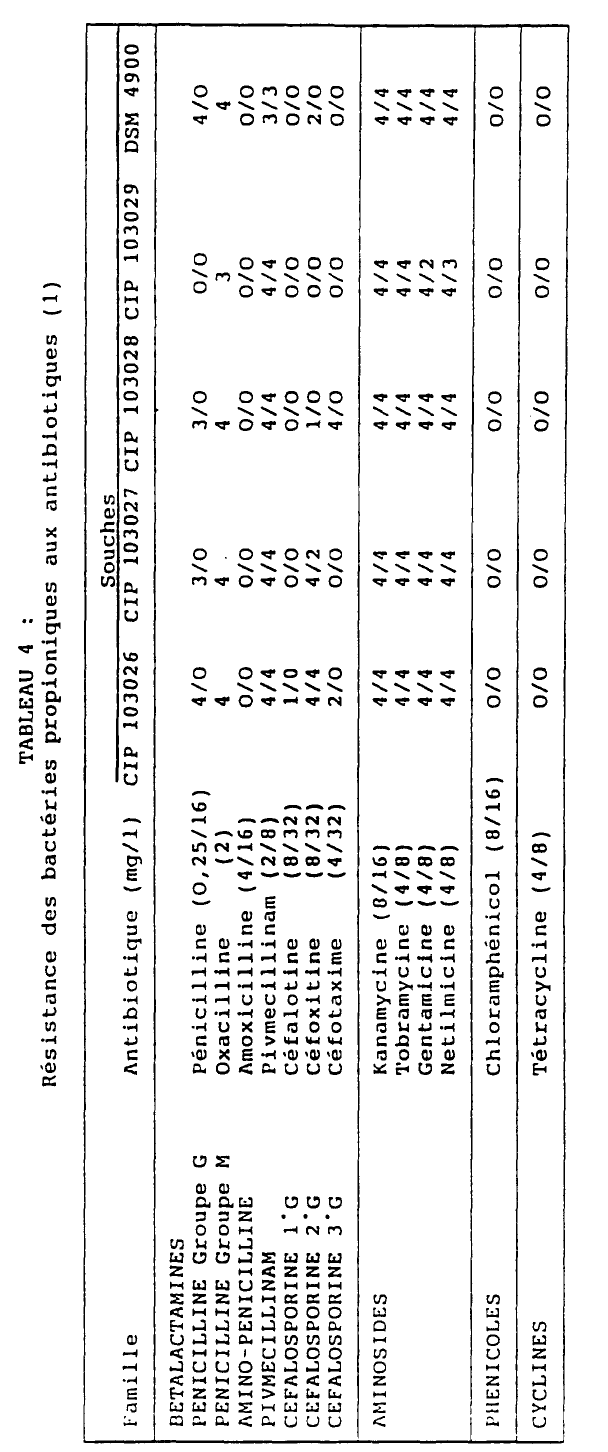

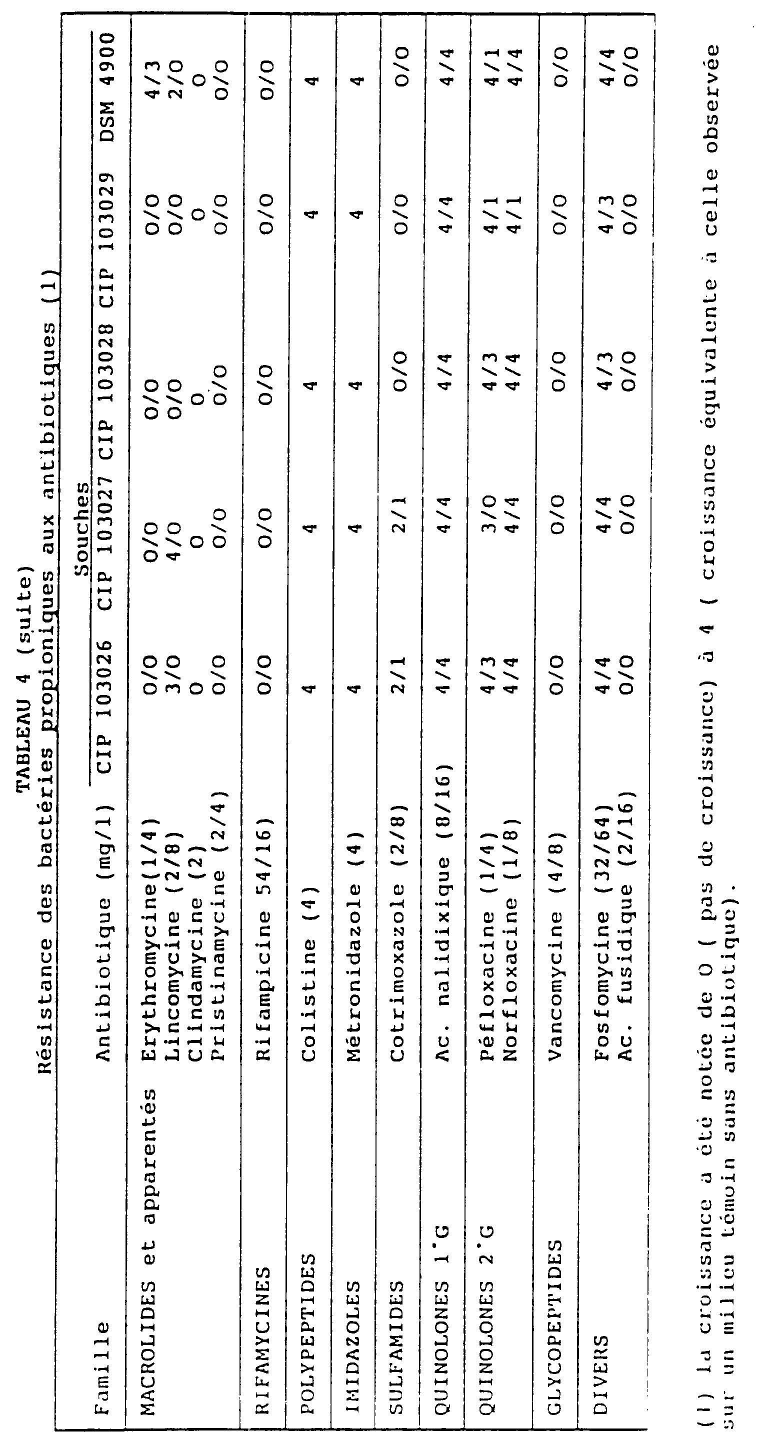

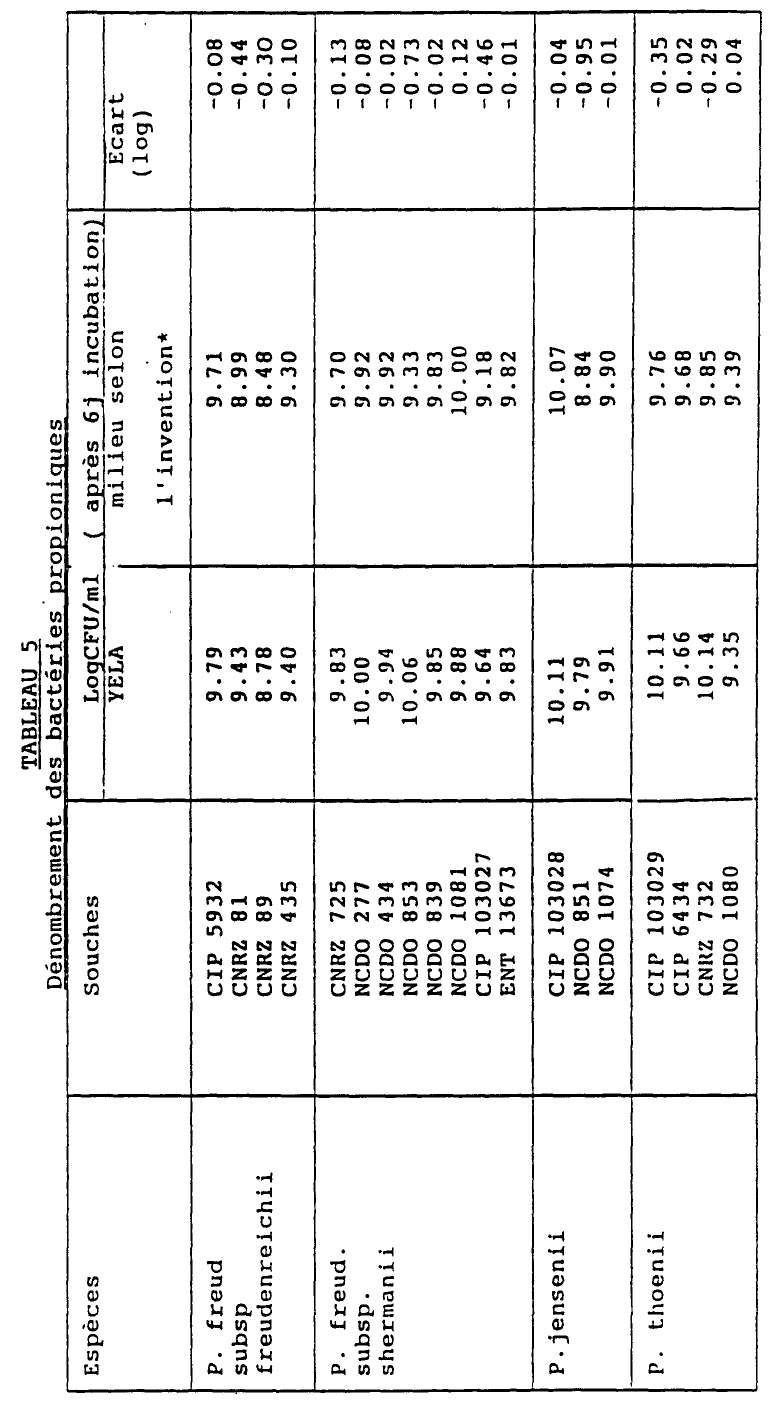

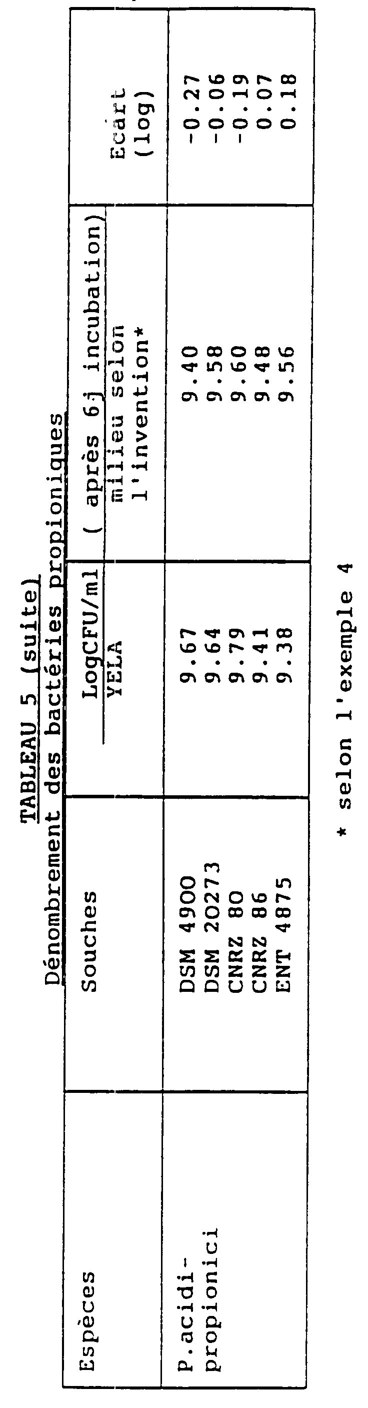

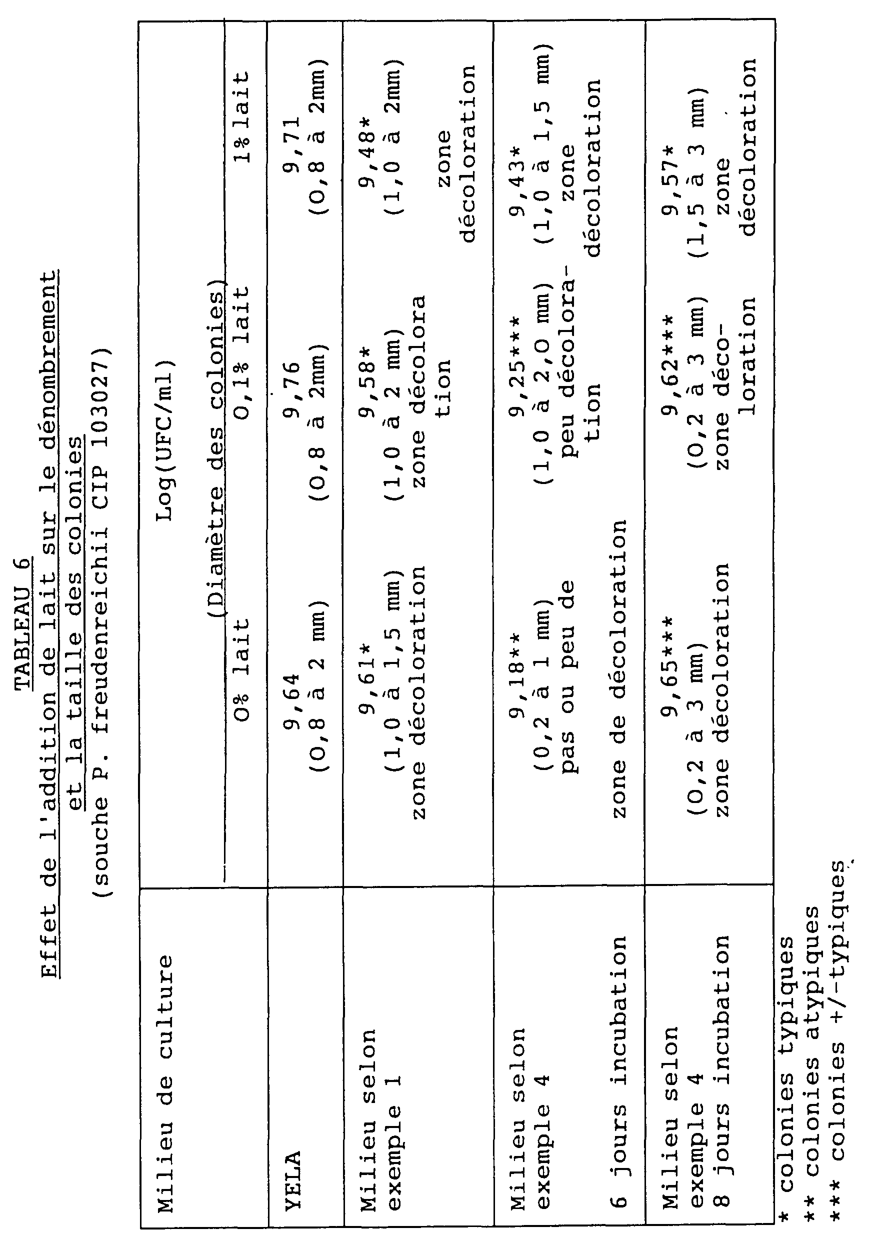

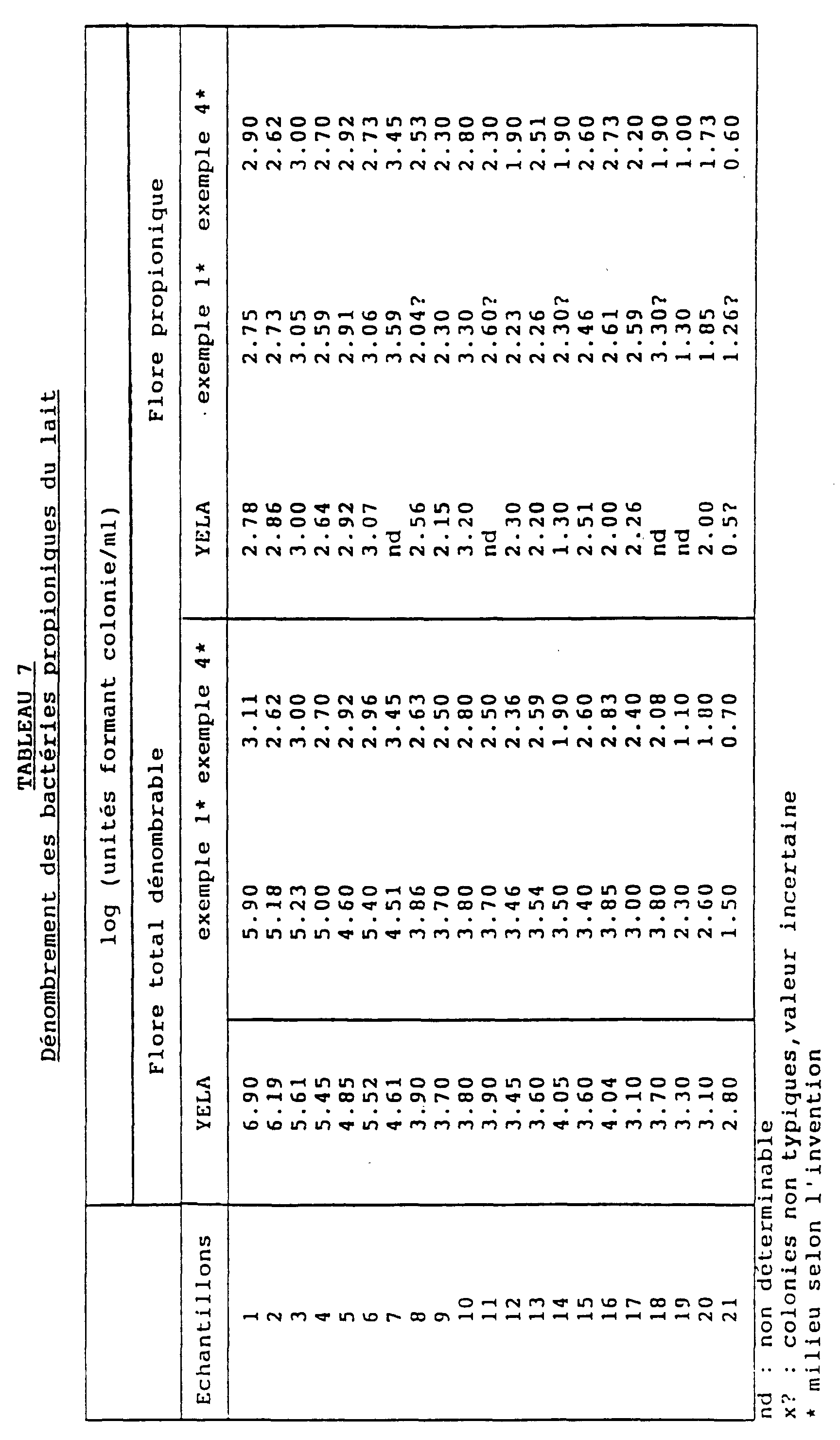

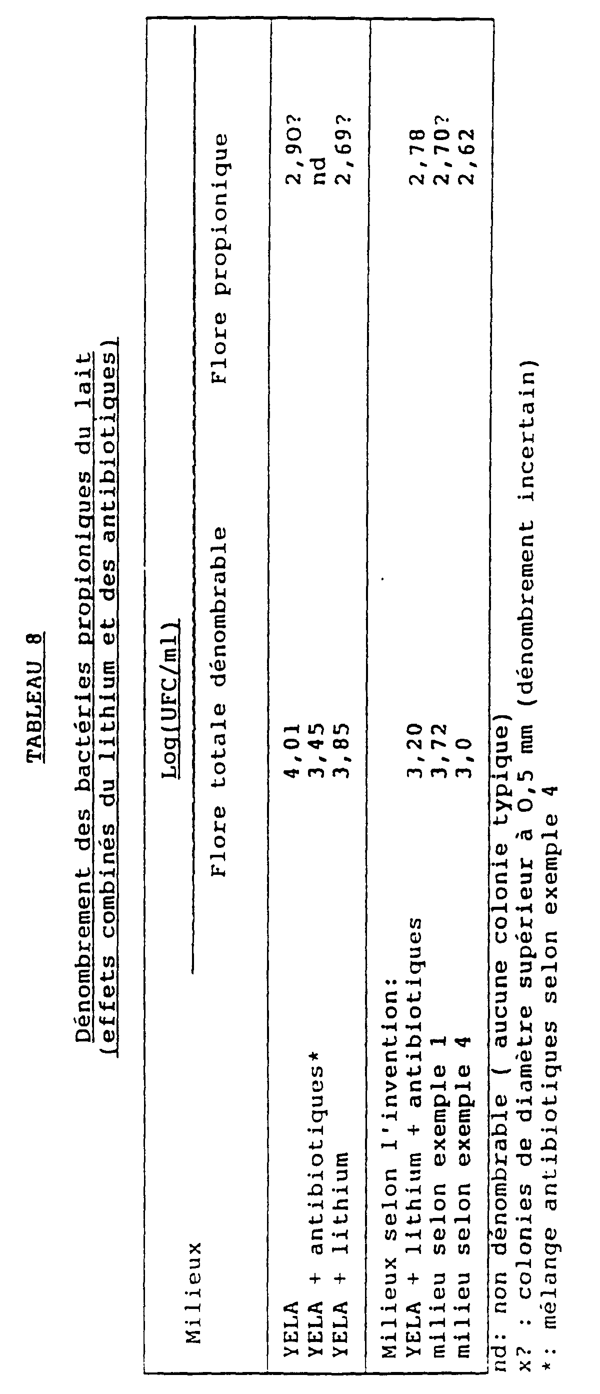

## Revendications

1. Milieu pour le dénombrement en anaérobiose de bactéries propioniques **caractérisé en ce qu'**il comprend un milieu complexe complémenté avec d'une part au moins un composé de lithium et d'autre part du glycérol ou de l'érythritol, et/ou un ou plusieurs antibiotiques choisis parmi la fosfomycine, des aminosides, des polypeptides, des quinolones de première et de seconde générations ou des imidazoles, auxquels les bactéries propioniques sont résistantes.

2. Milieu selon la revendication 1, **caractérisé en ce que** le milieu complexe est composé d'un mélange d'hydrolysat de caséine et d'un extrait de levure .

3. Milieu selon l'une des revendications 1 et 2, **caractérisé en ce qu'**il est gélosé.

4. Milieu selon l'une des revendications 1 à 3, **caractérisé en ce qu'**il comprend de 0,2 % à 2 % en poids du milieu de composé de lithium et de 0,01 % à 2 % en poids du milieu de glycérol ou d'érythritol.

5. Milieu selon l'une des revendications 1 a 4, **caractérisé en ce que** le composé de lithium est le lactate de lithium, l'hydroxyde de lithium ou le chlorure de lithium.

6. Milieu selon la revendication 5, **caractérisé en ce qu'**il comprend entre 2 et 20 g /l , préférentiellement de 5 à 15 g/l et encore plus préférentiellement environ 10g/l de lactate de lithium.

7. Milieu selon la revendication 6, **caractérisé en ce qu'**il comprend entre 0,1 et 20 g/l, préférentiellement entre 2 et 10, et encore plus préférentiellement environ 6 g/l de glycérol.

8. Milieu selon l'une des revendications 1 à 7, **caractérisé en ce qu'**il est composé environ de :
- 10 g de tryptone
- 10 g d'extrait de levure
- 10 g de lactate de lithium
- 0,25 g de PO₄HK₂
- 0,05 g de SO₄Mn
- 6 g de glycérol
- 15 g d'agar
- 0,05 g de pourpre de bromocrésol
- 1000 ml d'eau
le pH du milieu étant ajusté à 7.

9. Milieu selon l'une des revendications 1 à 8 **caractérisé en ce que** la concentration de chaque antibiotique dans le milieu est comprise entre 2 et 500 mg/l.

10. Milieu selon l'une des revendications 1 à 9, **caractérisé en ce qu'**il comprend entre 0,05% et 5% de lait.

11. Procédé de dénombrement de bactéries propioniques dans un échantillon biologique , **caractérisé en ce que** l'on incube sous anaérobiose ledit échantillon ou ses dilutions décimales dans un milieu selon l'une des revendications 1 à 10 et **en ce qu'**on effectue de manière connue le comptage desdites bactéries.

## Claims

1. Medium for the counting under anaerobic conditions of propionic bacteria **characterised in that** it comprises a complex medium supplemented with on the one hand at least one lithium compound and on the other glycerol or erythritol and/or one or more antibiotics selected from fosfomycin, aminosides, polypeptides, first or second generation quinolones or imidazoles to which the propionic bacteria are resistant.

2. Medium according to claim 1, **characterised in that** the complex medium comprises a mixture of a casein hydrolysate and a yeast extract.

3. Medium according to one of claims 1 and 2, **characterised in that** it is in agar form.

4. Medium according to one of claims 1 to 3, **characterised in that** it contains from 0.2 % to 2 % by weight of the medium of a lithium compound and from 0.01 % to 2 % by weight of the medium of glycerol or erythritol.

5. Medium according to one of claims 1 to 4, **characterised in that** the lithium compound is lithium lactate, lithium hydroxide or lithium chloride.

6. Medium according to claim 5, **characterised in that** it contains between 2 and 20 g/l, preferably between 5 and 15 g/l and even more preferably about 10 g/l of lithium lactate.

7. Medium according to claim 6, **characterised in that** it contains between 0.1 to 20 g/l, preferably from 2 to 10 g/l and even more preferably about 6 g/l of glycerol.

8. Medium according to one of claims 1 to 7, **characterised in that** it comprises about:
- 10 g of tryptone
- 10 g of yeast extract
- 10 g of lithium lactate
- 0.25 g of K₂HPO₄
- 0.05 g of MnSO₄
- 6 g of glycerol
- 15 g of agar
- 0.05 g of bromocresol purple
- 1 000 ml of water
the pH of the medium being adjusted to 7.

9. Medium according to one of claims 1 to 8, **characterised in that** the concentration of each antibiotic into the medium is between 2 and 500 mg/l.

10. Medium according to one of claims 1 to 9, **characterised in that** it contains between 0.05 % and 5 % of milk.

11. Method for counting propionic bacteria in a biological sample, **characterised in that** said sample or decimal dilutions of it are incubated anaerobically in a medium according to one of claims 1 to 10 and **in that** said bacteria are then counted by known methods.

## Patentansprüche

1. Milieu zur anaerob erfolgenden Zählung von Propiobakterien, **dadurch gekennzeichnet, dass** es ein komplexes Milieu umfasst, das einerseits durch mindestens eine Lithiumverbindung und andererseits durch Glycerin oder Erythrit und/oder ein oder mehrere, aus Fosfomycin, Aminosiden, Polypeptiden, Chinolonen der ersten und zweiten Generation oder Imidazolen ausgewählte Antibiotika, gegen die die Propiobakterien resistent sind, ergänzt wird.

2. Milieu nach Anspruch 1, **dadurch gekennzeichnet, dass** das komplexe Milieu aus einer Mischung von Caseinhydrolysat und einem Hefeextrakt besteht.

3. Milieu nach einem der Ansprüche 1 und 2, **dadurch gekennzeichnet, dass** es sich um Gelose handelt.

4. Milieu nach einem der Ansprüche 1 bis 3, **dadurch gekennzeichnet, dass** es 0,2 % bis 2 % Lithium, bezogen auf das Gewicht des Mediums, und 0,01 % bis 2 % Glycerin und Erythrit, bezogen auf das Gewicht des Mediums, umfasst.

5. Milieu nach einem der Ansprüche 1 bis 4, **dadurch gekennzeichnet, dass** es sich bei der Lithiumverbindung um Lithiumlactat, Lithiumhydroxid oder Lithiumchlorid handelt.

6. Milieu nach Anspruch 5, **dadurch gekennzeichnet, dass** es zwischen 2 und 20 g/l, vorzugsweise 5 bis 15 g/l und noch mehr bevorzugt etwa 10 g/l Lithiumlactat umfasst.

7. Milieu nach Anspruch 6, **dadurch gekennzeichnet, dass** es zwischen 0,1 und 20 g/l, vorzugsweise zwischen 2 und 10 und noch mehr bevorzugt etwa 6 g/l Glycerin umfasst.

8. Milieu nach einem der Ansprüche 1 bis 7, **dadurch gekennzeichnet, dass** es etwa aus:
- 10 g Trypton
- 10 g Hefeextrakt
- 10 g Lithiumlactat
- 0,25 g K₂HPO₄
- 0,05 g MnSO₄
- 6 g Glycerin
- 15 g Agar
- 0,05 g Bromcresolpurpur
- 1000 ml Wasser
besteht, wobei der pH-Wert des Milieus auf 7 eingestellt ist.

9. Milieu nach einem der Ansprüche 1 bis 8, **dadurch gekennzeichnet, dass** die Konzentration eines jeden Antibiotikums im Milieu zwischen 2 und 500 mg/l beträgt.

10. Milieu nach einem der Ansprüche 1 bis 9, **dadurch gekennzeichnet, dass** es zwischen 0,05 % und 5 % Milch enthält.

11. Verfahren zur Zählung von Propiobakterien in einer biologischen Probe, **dadurch gekennzeichnet, dass** man die Probe oder deren Verdünnungen mit Verdünnungsfaktoren von 10 in einem Milieu nach einem der Ansprüche 1 bis 10 unter anaeroben Bedingungen inkubiert und die Zählung der Bakterien auf bekannte Weise durchführt.
